Europäisches Patentamt

European Patent Office

Office européen des brevets

(11) **EP 0 987 012 A1**

(12) **DEMANDE DE BREVET EUROPEEN**

(43) Date de publication:
22.03.2000 Bulletin 2000/12

(51) Int Cl.⁷: **A61K 7/48**

(21) Numéro de dépôt: **99402053.5**

(22) Date de dépôt: **12.08.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **18.09.1998 FR 9811694**

(71) Demandeur: **L'OREAL**
**75008 Paris (FR)**

(72) Inventeurs:
• **Lemann, Patricia**
  **94000 Creteil (FR)**
• **De La Poterie, Valérie**
  **77820 Le Chatelet en Brie (FR)**

(74) Mandataire: **Lhoste, Catherine**
  **L'OREAL-DPI**
  **6 rue Bertrand Sincholle**
  **92585 Clichy Cédex (FR)**

(54) **Composition cosmétique sous forme d'émulsion comprenant une dispersion de particules de polymères stabilisées en surface dans une phase grasse liquide**

(57) La présente invention se rapporte à une composition, notamment cosmétique, dermatologique, hygiénique ou pharmaceutique, pour le soin et/ou le maquillage de la peau, se présentant sous forme d'une émulsion comprenant une dispersion de particules de polymère, stabilisées en surface dans la phase grasse liquide de l'émulsion et éventuellement au moins une matière colorante, la quantité de polymère étant en quantité suffisante pour conférer à la composition des propriétés de sans transfert. Cette composition permet d'obtenir sur les lèvres et/ou la peau un film souple, non gras, ayant des propriétés de sans transfert remarquables, tout en étant d'un très grand confort.

L'invention se rapporte également à l'utilisation de cette dispersion de polymère dans une telle composition.

## Description

La présente invention a trait à une composition sous forme d'émulsion contenant un polymère dispersible dans une phase grasse, destinée en particulier aux domaines cosmétique, dermatologique, pharmaceutique et hygiénique. Plus spécialement, l'invention se rapporte à une composition sans transfert pour le soin et/ou le maquillage de la peau aussi bien du visage que du corps humain, des muqueuses comme les lèvres et l'intérieur des paupières inférieures, ou encore des fibres kératiniques comme les cils, les sourcils, et les cheveux.

Cette composition peut se présenter notamment sous forme de pâte ou de crème plus ou moins fluide comme les fonds de teint ou rouges à lèvres fluides, les eyes liners, les mascaras, les compositions de protection solaire, de bronzage artificiel de la peau ou de maquillage du corps mais aussi les fards à paupières ou à joues. Cette composition présente des propriétés de sans transfert associées à un effet fraîcheur et à un confort amélioré par rapport aux produits connus.

Les produits de maquillage ou de soin de la peau ou des lèvres des êtres humains comme les fonds de teint ou les rouges à lèvres contiennent généralement des phases grasses telles que des cires et des huiles, des pigments et/ou charges et, éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

Depuis plusieurs années, les cosméticiens se sont intéressés aux compositions de rouge à lèvres et plus récemment aux compositions de fond de teint "sans transfert". Ainsi, la société Shiseido a envisagé dans sa demande de brevet JP-A-61-65809 des compositions de rouge à lèvres "sans transfert" contenant une résine siloxysilicate (à réseau tridimensionnel), une huile de silicone volatile à chaîne silicone cyclique et des charges pulvérulentes. De même la société Noevier a décrit dans le document JP-A-62-61911 des compositions de rouge à lèvres, d'eye liner, de fonds de teint "sans transfert" comportant une ou plusieurs silicones volatiles associées à une ou plusieurs cires hydrocarbonées.

Ces compositions, bien que présentant des propriétés de "sans transfert" améliorées ont l'inconvénient de laisser sur les lèvres, après évaporation des huiles de silicone, un film qui devient inconfortable au cours du temps (sensation de dessèchement et de tiraillement), écartant un certain nombre de femmes de ce type de rouge à lèvres. Pour améliorer le confort de ce type de composition, on pourrait leur ajouter des huiles non volatiles siliconées ou non, mais dans ce cas particulier, on perdrait en efficacité "sans transfert".

Plus récemment, la société Procter & Gamble a envisagé dans sa demande de brevet WO-A-96/36323 des compositions de mascara de type émulsion eau-dans-huile présentant une longue tenue, une résistance à l'eau et ne laissant pas de traces. Ces compositions contiennent, entre autre, une dispersion aqueuse de polymère, appelée généralement un latex, associée à un tensioactif du type alkyl ou alcoxy diméthicone copolyol, des huiles hydrocarbonées, des pigments et des charges ainsi que des cires. Les propriétés de sans transfert des films déposés ne sont pas parfaites. En particulier, une pression ou un frottement prononcé, conduit à une diminution de la couleur du dépôt et à un redépôt sur le support mis en contact avec ces films.

Il est, en outre connu des documents EP-A-497144 et FR-A-2 357 244 des compositions anhydres dites "sans transfert", contenant un polymère bloc styrène-éthylène-propylène associé à des cires, des huiles légères ou volatiles et des pigments. Ces compositions présentent l'inconvénient d'être peu confortables, d'avoir des propriétés cosmétiques quelconques, d'êtres grasses et d'être difficilement formulables. Par ailleurs, les propriétés "sans transfert" de ces compositions sont très moyennes.

Plus récemment, on a envisagé dans le document EP-A-775483 des compositions pour les lèvres sous forme de dispersion aqueuse de polymère filmogène. Ces compositions présentent des propriétés de "sans transfert" remarquables et conduisent à des films brillants, très appréciés par les consommateurs. Malheureusement, ces films présentent l'inconvénients d'être inconfortables au cours du temps, après évaporation de l'eau.

Il subsiste donc le besoin d'une composition ne présentant pas les inconvénients ci-dessus, et ayant notamment des propriétés de "sans transfert" remarquables, même lors d'une pression ou d'un frottement prononcé, un aspect plus ou moins brillant, adapté au désir de la consommatrice, ne desséchant pas la peau ou les lèvres sur lesquelles elle est appliquée, aussi bien lors de l'application qu'au cours du temps.

La demanderesse a constaté, de façon tout à fait surprenante, que l'utilisation d'une émulsion contenant dans la phase grasse liquide un polymère dispersible permettait d'obtenir une composition cosmétique, dermatologique, pharmaceutique ou hygiénique conduisant à un dépôt cohésif, de très bonne tenue, ne transférant pas du tout, résistant à l'eau, tout en étant très agréable à l'application et à porter tout au long de la journée. Le dépôt est notamment ni gras, ni sec, flexible et non collant. En outre la composition de l'invention présente des propriétés de confort à l'application et au cours du temps et notamment des propriétés de fraîcheur à l'application, d'hydratation et d'absence de sensations de tiraillement.

La présente invention a donc pour objet une composition à application topique, comprenant une phase grasse liquide et une phase aqueuse, l'une des phases étant dispersée dans l'autre phase, caractérisée par le fait qu'elle comprend, de plus, des particules de polymère stabilisées en surface dans ladite phase grasse liquide, ce polymère étant en quantité suffisante pour conférer des propriétés de sans transfert à ladite composition.

La quantité de polymère doit être suffisante pour former sur la peau et/ou les lèvres et/ou les fibres kératiniques un film apte à piéger les matières colorantes et/ou les actifs cosmétiques ou dermatologiques et/ou les huiles en vue de limiter, voire supprimer, leur transfert sur un support avec lequel le film est mis en contact. La quantité de polymère est fonction de la quantité de matières colorantes et/ou d'actifs et/ou d'huiles, contenus dans la composition. En pratique, la quantité de polymère est supérieure à 2 % en poids (en matière active), par rapport au poids total de la composition.

La composition selon l'invention est une dispersion homogène d'une phase grasse liquide dans une phase continue aqueuse ou une dispersion homogène d'une phase aqueuse dans une phase continue grasse liquide, la dispersion étant assurée par tout moyen et notamment à l'aide d'émulsionnant, de particules, ou de vésicules. Plus spécialement, la composition est une émulsion huile-dans-eau (H/E) ou eau-dans-huile (E/H). Elle peut être aussi une émulsion multiple et notamment une émulsion triple.

Cette composition est en particulier une composition cosmétique, dermatologique, hygiénique ou pharmaceutique. Elle contient donc des ingrédients compatibles avec la peau, les muqueuses et les fibres kératiniques.

Un autre objet de l'invention est l'utilisation dans une composition cosmétique ou pour la fabrication d'une composition dermatologique sous forme de dispersion d'une première phase dans une seconde phase, l'une des première et seconde phases étant une phase grasse liquide, d'une quantité suffisante de particules de polymère stabilisées en surface dans ladite phase grasse liquide, pour diminuer, voire supprimer, le transfert d'un film de composition déposé sur des muqueuses comme les lèvres et/ou sur la peau d'être humain et/ou les fibres kératiniques vers un support mis en contact avec le film.

Avantageusement, la composition contient au moins un ingrédient choisi parmi les actifs cosmétiques, dermatologiques, hygiéniques et pharmaceutiques, les matières colorantes et leurs mélanges. Grâce à la dispersion de particules de polymère stabilisées en surface présente dans la phase grasse liquide, la composition de l'invention permet de limiter, voire supprimer le transfert de la composition et en particulier le transfert des actifs et/ou des matières colorantes et donc de maintenir ces actifs et/ou matières colorantes là où elles sont été déposées.

L'invention a encore pour objet un procédé de soin cosmétique ou de maquillage des lèvres et/ou de la peau et/ou des fibres kératiniques, consistant à appliquer sur respectivement les lèvres et/ou la peau et/ou les fibres une composition cosmétique telle définie précédemment.

L'invention a encore pour objet un procédé cosmétique pour limiter, voire supprimer, le transfert d'une composition de maquillage ou de soin de la peau et/ou des lèvres et/ou des fibres kératiniques sur un support différent de la peau et/ou des lèvres et/ou des fibres, la dite composition étant sous forme de dispersion d'une première phase dans une seconde phase, l'une des phase étant une phase grasse liquide, consistant à introduire dans la phase grasse liquide une quantité suffisante de particules de polymère stabilisées en surface dans ladite phase grasse liquide.

Le polymère utilisé dans la présente demande peut être de toute nature. On peut ainsi employer un polymère radicalaire, un polycondensat, voire un polymère d'origine naturelle et leurs mélanges. Le polymère peut être choisi par l'homme du métier en fonction de ses propriétés et selon l'application ultérieure souhaitée pour la composition. De préférence le polymère utilisé est filmifiable. Il est toutefois possible d'utiliser un polymère non filmifiable.

Par polymère non filmifiable, on entend un polymère non capable de former, seul, un film isolable. Ce polymère permet, en association avec un composé non volatil du type huile, de former un dépôt continue et homogène sur la peau et les muqueuses.

De façon avantageuse, le polymère se présente sous forme de particules dispersées et stabilisées en surface par au moins un stabilisant.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

On a de plus constaté que les compositions selon l'invention, présentent des qualités d'étalement et d'adhésion sur la peau ou les muqueuses particulièrement intéressantes, ainsi qu'un toucher onctueux et agréable. Ces compositions ont, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte supplémentaire pour l'utilisatrice.

Les compositions selon l'invention comprennent donc avantageusement une dispersion stable de particules généralement sphériques d'au moins un polymère, dans une phase grasse liquide physiologiquement acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère mou à un polymère plus ou moins dur, permettant de régler les propriétés mécaniques de la composition en fonction de l'application envisagée.

Les polymères utilisables dans la composition de l'invention ont de préférence un poids moléculaire de l'ordre de 2000 à 10 000 000 et une (Tg) de -100°C à 300°C et mieux de - 10 à 50°C.

Il est possible d'utiliser des polymères filmifiables, de préférence ayant une (Tg) basse, inférieure ou égale à la température de la peau, notamment inférieure à 40°C. On obtient ainsi une dispersion qui peut filmifier lorsqu'elle est appliquée sur un support, ce qui n'est pas le cas lorsque l'on utilise des dispersions de pigments minéraux selon l'art antérieur.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à 40°C et notamment allant de - 10 à 30°C.

Parmi les polymères non filmifiables, on peut citer des homopolymères ou copolymères radicalaires, vinyliques ou acryliques, éventuellement réticulés, ayant de préférence une Tg supérieure ou égale à 40°C et notamment allant de 45 à 150°C.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tes que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth) acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth) acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et le lauryle. Comme (méth) acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymère d'acide (méth)acrylique et de (méth)acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth)acrylamides, en particulier d'alkyl en $C_2$-$C_{12}$ tels que le N-éthyl acrylamide, le N-t-butyl acrylamide, le N-octyl acrylamide ; les N- dialkyl ($C_1$-$C_4$) (méth) acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :

- la N-vinylpyrrolidone, la vinylcaprolactame, les vinyl N-alkyl($C_1$-$C_6$) pyrroles, les vinyloxazoles, les vinylthiazoles, les vinylpyrimidines, les vinylimidazoles,
- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol ou le phtalate de diallyle.

De façon non limitative, les polymères de l'invention peuvent être choisis parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

La phase grasse liquide dans laquelle est dispersé le polymère, peut être constituée de toute huile cosmétiquement ou dermatologiquement acceptable, et de façon plus générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Par "phase grasse liquide", on entend tout milieu non aqueux liquide à température ambiante (25°C) et pression atmosphérique.

Comme phase grasse liquide utilisable dans l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxanes (PDMS), éventuellement phénylés telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

On peut éventuellement utiliser une ou plusieurs huiles volatiles à température ambiante. Par "phase huile volatile", on entend tout milieu non aqueux susceptible de s'évaporer de la peau ou des lèvres ou des fibres, à température ambiante. Cette phase volatile comporte en particulier des huiles ayant une pression de vapeur à température ambiante et pression atmosphérique allant de $10^{-3}$ à 300mm de Hg (0,13 Pa à 40 000 Pa). Ces huiles volatiles facilitent, notamment, l'application de la composition sur la peau, les muqueuses, les fibres kératiniques. Ces huiles peuvent être des huiles hydrocarbonées, des huiles siliconées comportant éventuellement des groupements alkyle ou alkoxy pendants ou en bout de chaîne siliconée, les huiles fluorées.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone ainsi que les isoparaffines en $C_8$-$C_{16}$. Ces huiles volatiles représentent notamment de 5 à 97,5 % du poids total de la composition, et mieux de 20 à 75 %. Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETYLs et notamment l'isododécane.

Dans un mode particulier de réalisation de l'invention, on choisit le phase grasse liquide dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global 6 global selon l'espace de solubilité de HANSEN est défini dans l'article "Solubility parameter values" de Eric A. Grulke de l'ouvrage "Polymer Handbook" $3^{ème}$ édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN inférieur ou égal à 17 $(MPa)^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère et/ou de la nature du stabilisant, comme indiqué ci-après.

De plus, la phase grasse liquide totale dans laquelle est dispersé le polymère peut représenter de 5 % à 97,5 % du poids total de la composition et de préférence de 20 à 75 %. La partie non volatile représente au minimum 0 % et en pratique de 1 à 50 % du poids total de la composition. Cette phase grasse contient une ou plusieurs huiles compatibles entre elles.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie contient une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly 1 2(hydroxystéarique).

Ainsi, on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly

alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés, on peut citer ceux comprenant au moins un bloc résultant de la polymérisation d'au moins un monomère éthylénique, à une ou plusieurs liaisons éthyléniques éventuellement conjugués comme l'éthylène ou les diènes tels que le butadiène et l'isoprène, et au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène, polystyrène/polybutadiène tels que ceux vendus sous le nom de 'LUVITOL HSB' par BASF, du type polystyrène/copoly (éthylène-propylène) tels que ceux vendus sous le nom de 'KRATON' par Shell Chemical Co ou encore du type polystyrène/copoly(éthylène-butylène). Ces copolymères sont généralement appelés des copolymères de diène hydrogéné ou non.

Comme copolymères blocs greffés ou séquencés comprenant au moins un bloc résultant de la polymérisation d'au moins un monomère éthylénique à une ou plusieurs liaisons éthyléniques, éventuellement conjuguées tel que l'éthylène ou l'isobutylène, et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques éventuellement conjuguées comme l'éthylène ou les diènes, et d'au moins un bloc d'un polyéther tel qu'un polyalkylène en $C_2$-$C_{18}$ notamment un polyoxyéthylène et/ou polyoxypropylène, on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

D'autre part, lorsque la phase grasse liquide comprend au moins une huile de silicone, l'agent stabilisant est de préférence choisi dans le groupe constitué par les copolymères blocs greffés ou séquencés comprenant au moins un bloc de type polyorganosiloxane résultant de la polymérisation d'un siloxane et au moins un bloc d'un polymère radicalaire ou d'un polyéther ou d'un polyester comme les blocs polyoxypropyléné et/ou oxyéthyléné. Bien vérifier

Lorsque le phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au moins un monomère éthylénique à une ou plusieurs liaisons éthyléniques éventuellement conjuguées,

et au moins un bloc d'un polymère vinylique ou acrylique ou d'un polyéther ou d'un polyester, ou leurs mélanges.

De préférence, on utilise des polymères dibloc.

Les dispersions obtenues selon l'invention peuvent alors être utilisées dans une composition notamment cosmétique, dermatologique, pharmaceutique et/ou hygiénique, telle qu'une composition de soin ou de maquillage de la peau ou des lèvres, ou encore une composition capillaire ou une composition solaire ou de bronzage artificiel de la peau.

Suivant l'application, on pourra choisir d'utiliser des dispersions de polymères filmifiables ou non filmifiables, dans des huiles volatiles ou non volatiles.

Selon l'invention, la phase grasse liquide décrite ci-dessus peut constituer la phase continue de la composition ou bien la phase dispersée. L'autre phase est une phase aqueuse contenant de l'eau et éventuellement des solvants hydrosolubles comme des alcools inférieurs. Dans ce cas, ces solvants ne pourront pas constituer la phase grasse liquide. En effet pour former la composition de l'invention, les deux phases doivent être non miscibles. Avantageusement, la composition de l'invention est du type E/H ou H/E.

Ces émulsions peuvent être obtenues en utilisant un tensioactif ou un mélange de tensioactifs dont le HLB (balance hydrophile/lipophile) est adapté au sens de l'émulsion.

Comme tensioactif utilisable dans l'invention, adapté à l'obtention d'une émulsion E/H on peut citer ceux ayant un HLB inférieur 7 et notamment les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone. Comme tensioactif utilisable dans l'invention pour l'obtention d'une émulsion H/E on peux citer ceux ayant un HLB supérieur à 7 comme les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols. De façon générale, on peut utiliser tout tensioactif ionique (cationique ou anionique) amphotère et tout tensioactif non ionique, bien connu de l'homme du métier.

La composition peut comprendre avantageusement une ou plusieurs matières colorantes contenant un ou plusieurs composés pulvérulents et/ou un ou plusieurs colorants liposolubles et/ou hydrosolubles, par exemple à raison de 0,01 à 70% du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 50 % du poids total de la composition et mieux de 1 à 30%. Plus la quantité de composés pulvérulents diminue, plus les qualités de sans transfert et de confort augmentent. Le fait que les propriétés de sans transfert augmentent au fur et à mesure que la quantité de composés pulvérulents diminue est tout à fait surprenant. En effet, jusqu'à ce jour les propriétés de sans transfert des compositions de l'art antérieur augmentaient avec la quantité de composés pulvérulents. Inversement, leurs inconforts et leur sécheresse sur la peau ou muqueuses augmentaient.

Par ailleurs, la propriété de sans transfert augmente avec la quantité de polymère dispersible dans la phase grasse liquide. En pratique, le polymère peut représenter en matière active jusqu'à 60 % (en matière active ou sèche) du poids total de la composition. En utilisant au-dessus de 12 % en poids, de matière active de polymère et d'huile non volatile, dans la composition, on obtient un film sans transfert total. Entre 2 % et 12 % l'effet sans transfert est notable sans toutefois être total. On peut donc adapter les propriétés de sans transfert à volonté, ce qui n'était pas possible avec les compositions sans transfert de l'art antérieur, sans nuire au confort du film déposé.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon, de poly-$\beta$-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydrocarbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le $\beta$-carotène, l'huile de soja, le brun Soudan, le DC Yellow, 11, le DC Violet 2, le DC orange 5, le jaune quinoléine. Ils peuvent représenter de 0,01 à 20 % du poids de la compositions et mieux de 0,1 à 6 %. Les colorants hydrosolubles sont par exemple le jus de betterave, le bleu de méthylène le carotène et peuvent représenter jusqu'à 6 % du poids total de la composition.

Le polymère de la composition de l'invention permet la formation d'un film sur la peau, les lèvres et/ou les muqueuses et/ou les fibres, formant un réseau, piégeant les matières colorantes (y compris les charges) et/ou les actifs. Selon la quantité relative de matières colorantes, utilisée par rapport à la quantité de polymère stabilisé, utilisée, il est possible d'obtenir un film plus ou moins brillant et plus ou moins sans transfert.

Comme actifs cosmétiques, dermatologiques, hygiéniques ou pharmaceutiques, utilisables dans la composition de l'invention, on peut citer les hydratants, vitamines, acides gras essentiels, sphingolipides, filtres solaires. Ces actifs sont utilisés en quantité habituelle pour l'homme et notamment à des concentrations de 0,001 à 20 % du poids total de la composition.

La composition selon l'invention peut, de plus, comprendre, selon le type d'application envisagée, les constituants classiquement utilisées dans les domaines considérés, qui sont présents en une quantité appropriée à la forme galénique souhaitée.

En particulier, elle peut comprendre, outre, la phase grasse liquide dans laquelle le polymère est stabilisé des phases grasses additionnelles qui peuvent être choisies parmi les cires, les huiles, les gommes et/ou les corps gras pâteux, d'origine végétale, animale, minérale ou de synthèse, voire siliconé, et leurs mélanges.

Parmi les cires solides à température ambiante, susceptibles d'être présentes dans la composition selon l'invention, on peut citer les cires hydrocarbonées telles que la cire d'abeilles, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch, les huiles hydrogénées, les esters gras et les glycérides concrets à 25°C. On peut également utiliser des cires de silicone, parmi lesquelles on peut citer les alkyl, alcoxy et/ou esters de polyméthylsiloxane. Les cires peuvent se présenter sous forme de dispersions stables de particules colloïdales de cire telles qu'elles peuvent être préparées selon des méthodes connues, telles que celles de "Microemulsions Theory and Practice", L. M. Prince Ed., Academic Press (1977), pages 21-32. Comme cire liquide à température ambiante, on peut citer l'huile de Jojoba.

Les cires peuvent être présentes à raison de 0-50% en poids dans la composition et mieux de 10 à 30 %.

La composition peut comprendre, en outre, tout additif usuellement utilisé dans de telles compositions, tel que des épaississants, des antioxydants, des parfums, des conservateurs, des polymères liposolubles comme les polyalkylènes, notamment le polybutène, les polyacrylates et les polymères siliconés compatibles avec la phase grasse ainsi que les dérivés de polyvinylpyrolidone. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'une émulsion simple ou multiple à phase continue huileuse ou aqueuse, de dispersion huileuse dans une phase aqueuse grâce à des vésicules contenant des lipides ioniques et/ou non ioniques. Elles peuvent, en outre, avoir l'aspect d'une crème plus ou moins fluide, d'une pâte plus ou moins visqueuse, d'une émulsion solide coulée dans un moule en forme de coupelle ou de stick. La viscosité dynamique de la composition peut être choisie dans une large gamme allant de 0,001 à 800 Pa.s mesurée à 25°C avec un viscosimètre équipé d'un mobile tournant à 100 tour/min.

La composition de l'invention peut être un produit de maquillage de la peau, des lèvres ou des fibres kératiniques comme les fonds de teint, les fard à joues ou à paupières, les mascaras, les eye-liners, les rouges à lèvres, les produits de maquillage du corps (tatouage semi-permanent). Ces produits peuvent en outre, contenir un ou plusieurs actifs cosmétiques ou dermatologiques en vue d'apporter une valence soin au maquillage.

Ces compositions à application topique peuvent, par ailleurs, constituer une composition cosmétique, dermatologique, hygiénique ou pharmaceutique de protection, de traitement ou de soin du visage, du cou, des mains ou du corps humains et par exemple constituer une crème de soin, un produit solaire ou de bronzage artificiel, un baume de soin ou de protection des lèvres, une pommade ou un onguent dermatologique.

L'invention est illustrée plus en détail dans les exemples suivants.

**Exemple 1 de dispersion de polymère**

On prépare une dispersion de copolymère non réticulé d'acrylate de méthyle et d'acide acrylique dans un rapport 85/15, dans de l'isododécane, selon la méthode de l'exemple 1 du document EP-A-749 746, en remplaçant l'heptane par de l'isododécane. On obtient ainsi une dispersion de particules de poly(acrylate de méthyle/acide acrylique) stabilisées en surface dans de l'isododécane par un copolymère dibloc séquencé polystyrène/copoly(éhylène-propylène) vendu sous le nom de KRATON G1701 (Shell), ayant un taux de matière sèche de 22,6% en poids et une taille moyenne des particules de 175 nm (polydispersité : 0,05) et une Tg de 20°C. Ce copolymère est filmifiable.

**Exemple 2 de dispersion de polymère**

Cette dispersion se distingue de celle de l'exemple 1 par l'emploi d'un diméthicone copolyol dibloc comme stabilisant vendu sous le nom DC3225C par la société Dow Corning.

**Exemple 3 de dispersion de polymère**

On prépare une dispersion de polyméthacrylate de méthyle réticulé par du diméthacrylate d'éthylène glycol, dans de l'isododécane, selon la méthode de l'exemple 2 du document EP-A-749 746, en remplaçant L'ISOPAR L par de l'isododécane. On obtient ainsi une dispersion de particules de polyméthacrylate de méthyle stabilisées en surface dans de l'isododécane par un copolymère dibloc séquencé polystyrène/copoly(éhylène-propylène) vendu sous le nom de KRATON G1701 (Shell), ayant un taux de matière sèche de 19,7% en poids et une taille moyenne des particules de 135 nm (polydispersité : 0,05) et une Tg de 100°C. Ce copolymère est non filmifiable à température ambiante.

**Exemple 4 de fond de teint H/E**

On prépare la composition suivante :

| | | |
|---|---|---|
| . | dispersion de l'exemple 1 | 16,90 g |
| . | huile d'amandes d'abricot | 2,54 g |
| . | conservateurs | 0,50 g |
| . | hydroxyde de sodium solide | 0,03 g |
| . | gomme de xanthane | 0,17 g |
| . | carbomère | 0,08 g |
| . | glycérine/carbomère/propylène glycol (*) | 4,22 g |
| . | glycérine | 5,91 g |
| . | acide stéarique | 0,25 g |
| . | alcool stéarylique | 0,42 g |
| . | stéarate de sucrose (**) | 1,10 g |
| . | stéarate de polyéthylène glycol (PEG 8 stéarate) | 1,10 g |
| . | pigments (***) | 10,00 g |
| . | eau qsp | 100 g |

Ce fond de teint est préparé de la façon suivante : incorporation des corps gras et des émulsionnants dans la dispersion de polymère à une température de 75 °C ; homogénéisation de la phase grasse ; incorporation de la phase grasse à la phase aqueuse sous agitation toujours à 75 °C ; maintient de l'agitation jusqu'au refroidissement complet de la composition.

Ce fond de teint est plus fluide que celui de l'exemple 5, plus frais, tout ayant des propriétés de sans transfert comparable.

**Exemple 5 de fond de teint E/H**

On prépare la composition suivante :

| | | |
|---|---|---|
| . | dispersion de l'exemple 1 | 20,60 g |

(*) Lubrajel MS de chez Guardian
(**) Tegosoft PSE 141G de chez Goldschmidt
(***) Même mélange que dans exemple 5

- cyclométhicone     4,00 g
- cyclopentasiloxane     5,00 g
- diméthicone copolyol dans cyclopentasiloxane (****)     1,80 g
- poudre de Nylon (charge)     8,00 g
- oxyde de fer jaune enrobé par du PDMS     1,45 g
- oxyde de fer brun-jaune     0,67 g
- oxyde de fer noir     0,25 g
- oxyde de titane     7,63 g
- conservateurs     0,40 g
- parfum     0,60 g
- sulfate de magnésium     0,70 g
- succinate d'isostéaryle de diglycéryle     0,60 g
- eau     qsp 100 g

On obtient un fond de teint qui peut être appliqué sur le corps, notamment sur le cou et le visage. Le maquillage est naturel, mat, résistant à l'eau et présente de très bonnes propriétés de sans transfert.

Ce fond de teint est fabriqué de la façon suivante : prédisperser les pigments dans le cyclométhicone ; homogénéiser la phase grasse (tensioactif + huile) à 40-50°C ; laisser refroidir ; ajouter la dispersion de l'exemple 1 ; ajouter les pigments ; puis ajouter l'ensemble de la phase aqueuse dans la phase grasse précédente, d'abord sous agitation lente puis à forte puissance pendant 10 min.

## Contre Exemple de fond de teint E/H

On prépare la composition suivante :

- cyclométhicone     3,65 g
- cyclopentasiloxane     21,40 g
- isododécane     4,55 g
- diméthicone copolyol dans cyclopentasiloxane (*)     6,00 g
- poudre de Nylon (charge)     8,00 g
- copolymère de VA/vinyl butyl benzoate/crotonate en dispersion aqueuse     20,0 g
- oxyde de fer jaune     1,45 g
- oxyde de fer brun-jaune     0,67 g
- oxyde de fer noir     0,25 g
- oxyde de titane     7,63 g
- conservateurs     0,40 g
- parfum     0,60 g
- sulfate de magnésium     0,60 g
- succinate d'isostéaryle diglycéryle     2,00 g
- adipate diisopropyle     1,00 g
- eau     qsp 100 g

Dans ce contre exemple, le copolymère et l'adipate de diisopropyle sont ajoutés à la fin, après émulsification.

Un test sensoriel a été effectué avec le fond de teint de l'exemple 5 et du contre exemple sur plusieurs

(****) Abil EM 97 de chez Goldschmidt

personnes. Le test de sans transfert a été réalisé dans les conditions suivantes : application du fond de teint sur le visage et le cou par demi-visage et demi-cou, après avoir appliqué une crème de soin hydratante "Hydrative" de chez Lancôme ; séchage à l'air libre pendant 10 minutes ; pose d'une collerette en tissu autour du cou pendant 30 minutes. Les propriétés de sans transfert sont notées de 0 à 7 ; plus le chiffre est élevé, plus le fond de teint transfère.

Le fond de teint de l'invention a reçu la note moyenne de 2 contre la note moyenne de 3,5 pour le fond de teint avec dispersion aqueuse de polymère. En outre, pour chaque personne du test, la formule de l'invention est toujours meilleure en sans transfert.

De plus les personnes du test ont jugé le produit de l'invention facile à étaler, doux, très glissant, conférant un maquillage homogène et adhérent, de couvrance légère. Le teint est unifié. La texture du produit est jugé fluide mais plus consistante que celle contenant la dispersion aqueuse de polymère, à l'application. Le démaquillage s'est effectué avec un démaquillant classique (Galateis de Chez Lancôme) sans laisser de traces .

## Exemple 6 de rouge à lèvres E/H

On prépare la composition suivante :

- dispersion de l'exemple 1     32,74 g
- oxyde de fer brun-jaune     4,00 g
- conservateurs     0,25 g
- sulfate de magnésium     0,82 g
- copolymère de vinylpyrrolidone/1-eicosène     0,44 g
- diméthicone copolyol à groupement $\alpha$-$\omega$ d'oxyde d'éthylène dans PDMS (****)     2,12 g . phényl triméthylsiloxy trisiloxane (20 cst à 20° C, PM 372)     0,76 g . mono, di glycérides d'acide isostéarique estérifés par acide succinique     0,71 g
- octyldodécanol     0,33 g
- eau     qsp 100 g

## Contre Exemple de rouge à lèvres

On prépare la composition suivante :

- dispersion aqueuse de polymère styrène acrylate (45 %)     50,00 g
- oxyde de fer brun-jaune     4,00 g
- conservateurs     0,25 g
- gélifiant acrylique     5,00 g
- cire polyéthylène/polytétrafluoroéthylène (50/50)     4,50 g
- alcool éthylique     5,00 g
- additifs     3,00 g
- eau     qsp 100 g

Un test sensoriel a été effectué avec le produit à lèvres de l'exemple 6 et du contre exemple de rouge à lèvres sur plusieurs personnes. Le test de sans transfert a été réalisé dans les conditions suivantes : application du rouge à lèvres par demi-lèvres ; séchage à l'air libre pendant 2 minutes ; évaluation du transfert par test du bisou sur papier filtre ; démaquillage avec un démaquillant classique (Galateisl de Chez Lancôme).

Le rouge de l'invention est ressorti avec de propriétés de sans transfert équivalentes à celles du rouge contenant la dispersion aqueuse de polymère mais avec des propriétés de confort améliorées (moins de tiraillement, une sensation de fraîcheur à l'application), et un démaquillage plus facile. Le produit de l'invention est, de plus, facile à maquiller.

**Exemple 7 d'eye-liner E/H**

On prépare la composition suivante :

.   dispersion de l'exemple 1        32,74 g
.   oxyde de fer noir       15,00 g
.   isoparaffine hydrogénée        0,77 g
.   sulfate de magnésium       0,82 g
.   copolymère             de         vinylpyrrolidone/ 1-eicosène      0,44 g
.   diméthicone copolyol à groupement α-ω d'oxyde d'éthylène dans PDMS (****)       2,12 g
.   phényl triméthylsiloxy trisiloxane (20 cst à 20° C, PM 372)       0,76 g
.   mono, di glycérides d'acide isostéarique estérifés par acide succinique       0,71 g
.   copolymère acide méthacrylique/méthacrylate de méthoxy polyéthylène glycol/méthacrylate de méthyle solubilisé dans eau/propylène glycol (i)       0,38 g
.   propylène glycol       4,81 g
.   octyldodécanol       0,33 g
.   eau       qsp 100 g

(i) Arlatone 3315 de Chez ICI (tensioactif)

Cet eye-liner a été testé sur paupière nue puis démaquillage avec deux démaquillants classiques Bifacil et Effacil de chez Lancôme. La texture est jugée fluide, glissante et adhérente, ne filante pas et rendant le tracé très précis et net, à la lisière et à la pointe du trait. On peut même repasser sur le trait après séchage. Le film est homogène. Le maquillage est doux et s'élimine facilement. Le confort est jugé bon.

**Revendications**

1.  Composition à application topique, comprenant une phase grasse liquide et une phase aqueuse, l'une des phases étant dispersée dans l'autre phase, caractérisée par le fait qu'elle comprend, de plus, des particules de polymère stabilisées en surface dans ladite phase grasse liquide, le polymère étant en quantité suffisante pour conférer des propriétés de sans transfert à la composition.

2.  Composition selon la revendication 1, dans laquelle le polymère se présente sous forme de particules dispersées et stabilisées en surface par au moins un stabilisant.

3.  Composition selon l'une des revendications précédentes, dans laquelle le polymère est choisi parmi les polymères radicalaires, les polycondensats, les polymères d'origine naturelle et leurs mélanges.

4.  Composition selon l'une des revendications précédentes, dans laquelle le polymère est choisi parmi les polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères ou copolymères acryliques et/ou vinyliques ; copolymères acryliques-silicone ; polyacrylamides ; polymères siliconés, polymères fluorés et leurs mélanges.

5.  Composition selon l'une des revendications précédentes, dans laquelle le polymère est filmifiable.

6.  Composition selon l'une des revendications précédentes, dans laquelle la phase grasse liquide est constitué d'huiles d'origine minérale, animale, végétale ou synthétique, carbonées, hydrocarbonées, fluorées et/ou siliconées, seules ou en mélange.

7.  Composition selon l'une des revendications précédentes, dans laquelle la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadéméthylcyclohexasiloxane, l'heptaméthylhexyltrisiloxane, l'heptaméthyloctyltrisiloxane ou les isoparaffines en $C_8$-$C_{16}$, et notamment l'isododécane.

**8.** Composition selon l'une des revendications précédentes, dans laquelle la phase grasse liquide est choisie dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

**9.** Composition selon l'une des revendications précédentes, caractérisée en ce que la phase grasse liquide contient au moins une huile volatile.

**10.** Composition selon l'une des revendications précédentes, dans laquelle la phase grasse liquide représente de 5 à 97,5 % du poids total de la composition.

**11.** Composition selon l'une des revendications 2 à 10, dans laquelle le stabilisant est choisi parmi les polymères séquencés, les polymères greffés, les polymères statistiques et leurs mélanges.

**12.** Composition selon la revendication 11, dans laquelle le stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide poly-12(hydroxystéarique); les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alkyle en $C_1$-$C_4$, ou d'acrylates ou de méthacrylates d'alkyle en $C_8$-$C_{30}$ ; les copolymères blocs greffés ou séquencés comprenant au moins un bloc résultant de la polymérisation de diène et au moins un bloc d'un polymère vinylique ; les copolymères blocs greffés ou séquencés comprenant au moins un bloc résultant de la polymérisation de diène et au moins un bloc d'un polymère acrylique ; les copolymères blocs greffés ou séquencés comprenant au moins un bloc résultant de la polymérisation de diène et au moins un bloc d'un polyéther.

**13.** Composition selon la revendication 11 ou 12, caractérisée en ce que le stabilisant est un polymère bloc greffé ou séquencé, comprenant au moins un bloc résultant de la polymérisation de diène et au moins un bloc d'un polymère vinylique ou comprenant un bloc polyoxypropyléné et/ou oxyéthyléné et un bloc résultant de la polymérisation d'un siloxane.

**14.** Composition selon l'une des revendications précédentes, comprenant, en outre, au moins une phase grasse additionnelle choisie parmi les cires, les gommes et/ou les corps gras pâteux, d'origine végétale, animale, minérale, de synthèse, ou siliconé, et leurs mélanges.

**15.** Composition selon l'une des revendications précédentes, comprenant, en outre, au moins une matière colorante.

**16.** Composition selon a revendication 15, caractérisée en ce que la matière colorante comprend des composés pulvérulents choisis parmi les charges et/ou les pigments et/ou les nacres.

**17.** Composition selon la revendication 16, caractérisée en ce que les composés pulvérulents représentent jusqu'à 50 % du poids total de la composition.

**18.** Composition selon l'une des revendications précédentes, caractérisée en ce que le polymère représente (en matière sèche) jusqu'à 60 % du poids total de la composition.

**19.** Composition selon l'une des revendications précédentes, se présentant sous forme d'une émulsion huile-dans-eau, eau-dans-huile, d'une émulsion multiple ou de dispersion d'huile-dans-eau grâce à des vésicules contenant des lipides ioniques et/ou non ioniques.

**20.** Composition selon l'une des revendications précédentes, se présentant sous la forme d'un produit de soin et/ou de maquillage de la peau et/ou des lèvres et/ou des fibres kératiniques.

**21.** Composition selon l'une des revendications précédentes, se présentant sous la forme d'un fond de teint, d'un fard à joues ou à paupières, d'un rouge à lèvres, d'un baume de soin ou de protection pour les lèvres, d'un mascara, d'un eye-liner, d'un maquillage du corps, d'une crème solaire ou de bronzage artificiel de la peau.

22. Composition selon l'une des revendications précédentes, caractérisé en ce qu'elle contient, en outre, au moins un actif cosmétique ou dermatologique.

23. Utilisation dans une composition cosmétique ou pour la fabrication d'une composition dermatologique sous forme de dispersion d'une première phase dans une seconde phase, l'une des première et seconde phases étant une phase grasse liquide, d'une quantité suffisante de particules de polymère stabilisées en surface dans ladite phase grasse liquide, pour diminuer, voire supprimer, le transfert d'un film de composition déposé sur des muqueuses comme les lèvres et/ou sur la peau d'être humain et/ou les fibres kératiniques vers un support mis en contact avec le film.

24. Procédé de soin cosmétique ou de maquillage des lèvres et/ou de la peau et/ou des fibres kératiniques, consistant à appliquer sur respectivement les lèvres et/ou la peau et/ou les fibres une composition cosmétique telle définie aux revendications 1 à 22.

25. Procédé cosmétique pour limiter, voire supprimer, le transfert d'une composition de maquillage ou de soin de la peau et/ou des lèvres et/ou des fibres kératiniques sur un support différent de la peau et/ou lèvres et/ou fibres, la dite composition étant sous forme de dispersion d'une première phase dans une seconde phase, l'une des phase étant une phase grasse liquide, consistant à introduire dans la phase grasse liquide une quantité suffisante de particules de polymère stabilisées en surface dans ladite phase grasse liquide.

**EP 0 987 012 A1**

**Office européen des brevets** | **RAPPORT DE RECHERCHE EUROPEENNE**

Numéro de la demande

EP 99 40 2053

## DOCUMENTS CONSIDERES COMME PERTINENTS

| Catégorie | Citation du document avec indication, en cas de besoin, des parties pertinentes | Revendication concernée | CLASSEMENT DE LA DEMANDE |
|---|---|---|---|
| D,A | EP 0 749 747 A (OREAL) 27 décembre 1996 (1996-12-27) --- | | A61K7/48 |
| D,A | EP 0 749 746 A (OREAL) 27 décembre 1996 (1996-12-27) --- | | |
| A | WO 98 38981 A (PENNZOIL PROD CO) 11 septembre 1998 (1998-09-11) --- | | |
| A | WO 94 12190 A (PENNZOIL PROD CO) 9 juin 1994 (1994-06-09) ----- | | |

| | DOMAINES TECHNIQUES RECHERCHES |
|---|---|
| | A61K |

Le présent rapport a été établi pour toutes les revendications

| Lieu de la recherche | Date d'achèvement de la recherche | Examinateur |
|---|---|---|
| LA HAYE | 17 septembre 1999 | Stienon, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

15

## ANNEXE AU RAPPORT DE RECHERCHE EUROPEENNE
## RELATIF A LA DEMANDE DE BREVET EUROPEEN NO.

EP 99 40 2053

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

17-09-1999

| Document brevet cité au rapport de recherche | | Date de publication | Membre(s) de la famille de brevet(s) | | | Date de publication |
|---|---|---|---|---|---|---|
| EP 0749747 | A | 27-12-1996 | FR | 2735689 | A | 27-12-1996 |
| | | | AT | 174502 | T | 15-01-1999 |
| | | | CA | 2197496 | A | 09-01-1997 |
| | | | DE | 69601147 | D | 28-01-1999 |
| | | | DE | 69601147 | T | 02-06-1999 |
| | | | ES | 2128149 | T | 01-05-1999 |
| | | | WO | 9700663 | A | 09-01-1997 |
| | | | JP | 10501005 | T | 27-01-1998 |
| | | | US | 5851517 | A | 22-12-1998 |
| EP 0749746 | A | 27-12-1996 | FR | 2735691 | A | 27-12-1996 |
| | | | FR | 2735690 | A | 27-12-1996 |
| | | | FR | 2735692 | A | 27-12-1996 |
| | | | FR | 2735684 | A | 27-12-1996 |
| | | | AT | 157529 | T | 15-09-1997 |
| | | | DE | 69600059 | D | 09-10-1997 |
| | | | DE | 69600059 | T | 05-02-1998 |
| | | | DK | 749746 | T | 23-02-1998 |
| | | | ES | 2110857 | T | 16-02-1998 |
| | | | WO | 9700662 | A | 09-01-1997 |
| | | | GR | 3025474 | T | 27-02-1998 |
| | | | JP | 10502389 | T | 03-03-1998 |
| WO 9838981 | A | 11-09-1998 | AU | 6539198 | A | 22-09-1998 |
| WO 9412190 | A | 09-06-1994 | CA | 2128423 | A | 09-06-1994 |
| | | | DE | 626855 | T | 03-08-1995 |
| | | | EP | 0626855 | A | 07-12-1994 |
| | | | GR | 95300037 | T | 30-06-1995 |
| | | | JP | 7504441 | T | 18-05-1995 |

EPO FORM P0460

Pour tout renseignement concernant cette annexe : voir Journal Officiel de l'Office européen des brevets, No.12/82